(19) Europäisches Patentamt
European Patent Office
Office européen des brevets

(11) Veröffentlichungsnummer: **0 206 270 B1**

(12) **EUROPÄISCHE PATENTSCHRIFT**

(45) Veröffentlichungstag der Patentschrift: **21.11.91**

(21) Anmeldenummer: **86108404.4**

(22) Anmeldetag: **20.06.86**

(51) Int. Cl.[5]: **C07D 217/14**, C07D 405/04, C07D 409/04, C07D 519/00, C07D 217/16, C07D 401/04, G03G 5/06

(54) **1,3,4-Tri-(het-)aryl-isochinoline, Verfahren zu ihrer Herstellung und ihre Verwendung als photoleitfähige Verbindungen.**

(30) Priorität: **28.06.85 DE 3523180**

(43) Veröffentlichungstag der Anmeldung: **30.12.86 Patentblatt 86/52**

(45) Bekanntmachung des Hinweises auf die Patenterteilung: **21.11.91 Patentblatt 91/47**

(84) Benannte Vertragsstaaten: **DE FR GB NL**

(56) Entgegenhaltungen: **EP-A- 0 092 255**

(73) Patentinhaber: **HOECHST AKTIENGESELLSCHAFT**
**Postfach 80 03 20**
**W-6230 Frankfurt am Main 80(DE)**

(72) Erfinder: **Pawlowski, Georg, Dr. Dipl.-Chem.**
**Blücherstrasse 48**
**W-6200 Wiesbaden(DE)**

**EP 0 206 270 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die vorliegende Verbindung betrifft neue 1,3,4-Tri-(het-) aryl-isochinoline, Verfahren zur Herstellung der Verbindungen und deren Verwendung als photoleitfähige Verbindungen, zum Beispiel in der Elektrophotographie oder der Xerographie.

Aus der Literatur sind bereits einige Verfahren zur Herstellung von 1,3,4-Triarylisochinolinen, insbesondere von 1,3,4-Triphenyl-isochinolin, bekannt.

So wurde 1,3,4-Triphenylisochinolin durch Entwässerung von Benzaminotriphenylethylalkohol mit Phosphorpentoxid hergestellt (W. Krabbe et al., Chem. Ber., 71, 64 (1938)). Die gleiche Verbindung wurde durch saure Zersetzung von Benzoazanorcaran erhalten (V. Nair, J. Org. Chem., 37, 2508 (1972)). Schließlich wurden 1,3,4-Triphenylisochinolin und einige weitere Derivate durch Umsetzung von 1,2-Dibenzoylbenzol mit entsprechenden Aminomethylaromaten unter basischen Kondensationsbedingungen synthetisiert (Sh. Mataka et al., Heterocycles, 14, 789 (1980)).

Der vorliegenden Erfindung liegt die Aufgabe zugrunde, neue Isochinolin-Verbindungen zu schaffen, die zum Beispiel als hochempfindliche Photoleiter eingesetzt werden können.

Gegenstand der vorliegenden Erfindung sind neue 1,3,4-Tri-(het-)arylisochinoline der allgemeinen Formel I

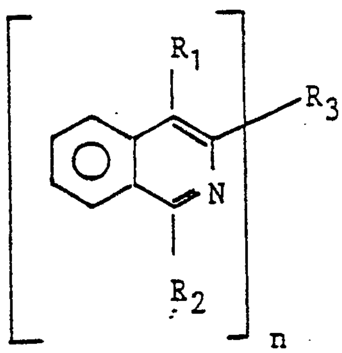

I

in der

$R_1$ und $R_2$ gleich oder verschieden sind und Di-($C_1$ bis $C_4$)alkylaminophenyl, Julolidyl oder Aminophenyl,

$R_3$ Phenyl, Naphthyl oder Phenylen, die gegebenenfalls durch mindestens ein Halogen, eine Nitro-, Mono- oder Di($C_1$ bis $C_4$)alkylamino-, ($C_1$ bis $C_4$)Alkyl- oder -Alkoxy-, Methylendioxy-, Amino($C_1$ bis $C_4$)alkyl- oder Sulfonamido-Gruppe substituiert sind, oder Furanyl, Thienyl, Pyridyl, Julolidyl oder Benzimidazolyl bedeuten, und

n gleich 1 oder 2 ist.

Bevorzugte Verbindungen sind solche der allgemeinen Formel I, in der

$R_1$ und $R_2$ gleich oder verschieden sind und 4-Di($C_1$ bis $C_4$)alkylaminophenyl,

$R_3$ Phenyl oder Naphthyl, die gegebenenfalls durch mindestens ein Halogen, eine Nitro-, Mono-oder Di($C_1$ bis $C_4$)alkylamino-, ($C_1$ bis $C_4$)Alkyl- oder -Alkoxy-, Methylendioxy-, Amino($C_1$ bis $C_4$)alkyl- oder Sulfonamido-Gruppe substituiert sind, oder Furanyl, Thienyl, Pyridyl, Julolidyl oder Benzimidazolyl

bedeuten und

n gleich 1 ist.

Insbesondere haben sich als Photoleiter Verbindungen der allgmeinen Formel I bewährt, bei denen

$R_1$ und $R_2$ 4-Dimethylaminophenyl oder 4-Diethylaminophenyl,

$R_3$ Phenyl oder Naphthyl, die gegebenenfalls durch mindestens ein Halogen, eine ($C_1$ bis $C_4$)Alkyl-, -Alkoxy-, Methylendioxy- oder Amino($C_1$ bis $C_4$)alkyl-Gruppe substituiert sind,

bedeuten und

n gleich 1 ist.

Gegenstand der vorliegenden Erfindung ist auch ein Verfahren zur Herstellung der Verbindungen nach der allgemeinen Formel I, bei dem man ein Diketon der allgemeinen Formel II

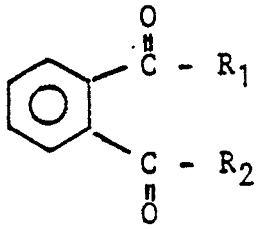

II

mit einer aromatischen oder heteroaromatischen Aminomethylverbindung der allgemeinen Formel III

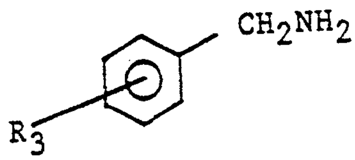

III

in denen $R_1$ $R_2$ und $R_3$ die angegebenen Bedeutungen besitzen, in einem Lösungsmittel oder Lösungsmittelgemisch unter Zusatz eines basischen Kondensationsmittels im Temperaturbereich zwischen 60 und 200 ° C umsetzt, das Reaktionsprodukt abtrennt, gegebenenfalls reinigt und trocknet.

Als Lösungsmittel verwendet man Ethanol, Ethylenglykolmonomethylether, Ethylenglykolmonoethylether, Butanol oder Dimethylformamid. Die Umsetzung wird vorzugsweise in Gegenwart eines Alkalialkoholates durchgeführt.

Die unter der Bedeutung von $R_1$, $R_2$ und $R_3$ in den allgemeinen Formeln I bis III aufgeführten Alkyl- oder Alkoxygruppen, sowie die sich hiervon ableitenden Gruppen enthalten erfindungsgemäß vorzugsweise 1 bis 4 Kohlenstoffatome.

Die erfindungsgemäßen Verbindungen werden hergestellt und aufgearbeitet, indem man ein geeignet substituiertes Diketon der allgemeinen Formel II mit einer Aminomethylverbindung der allgemeinen Formel III in einem Lösungsmittel unter Zusatz eines stark basischen Kondensationsmittels umsetzt.

Als Diketone der allgemeinen Formel II kommen Verbindungen wie 1,2-Bis(4-dimethylaminobenzoyl)-benzol oder 1-(4-Diethylaminobenzoyl)-2-(4-dimethylaminobenzoyl)-benzol in Betracht, die nach bekannten Verfahren (A. Guyot und A. Haller, A. CH. 19, 297 (1910)) hergestellt werden können. Andere, bisher nicht in der Literatur beschriebene Ketone der allgmeinen Formel II, wie beispielsweise 1-(4-Dimethylaminobenzoyl)-2-(4-morpholinobenzoyl)-benzol, lassen sich analog zu den literaturbekannten Verfahren herstellen.

Da die Herstellung der Diketone der Formel II nach diesen Methoden allerdings recht aufwendig und zeitraubend ist, wurde innerhalb der vorliegenden Erfindung ein vereinfachtes Verfahren zur Herstellung der gewünschten Ketone, wie beispielsweise 1,2-Bis(4-diethylaminobenzoyl)-benzol, bereitgestellt, das in Beispiel 1 beschrieben wird.

Als Aminomethylderivate der allgemeinen Formel III kommen in Betracht: Benzylamin, Chlorbenzylamine, Nitrobenzylamine, Methylbenzylamine, Methoxybenzylamine, Dialkylaminobenzylamine, Diarylaminobenzylamine, Aminomethylbenzylamine oder Aminomethylpyridine, Aminomethylthiophen, Aminomethylfuran oder Aminomethylbenzimidazol. Solche Verbindungen der allgmeinen Formel III sind in vielen Fällen kommerziell erhältlich oder nach zahlreichen bekannten Synthesemethoden zugänglich. Sind die zu verwendenden Aminomethylderivate der allgemeinen Formel III beispielsweise an der Luft zersetzlich, so lassen sich erfindungsgemäß auch deren Salze, im allgemeinen deren salz- oder schwefelsauren Salze, für die Umsetzung verwenden.

Die Umsetzung der Diketone der allgemeinen Formel II mit den Aminomethylderivaten der allgemeinen Formel III erfolgt bevorzugt in polar-protischen Lösungsmitteln oder Lösungsmittelgemischen. Als brauchbare Lösungsmittel haben sich Alkohole, Etheralkohole oder substituierte Formamide erwiesen. Es lassen sich auch Pyrrolidone oder gewisse Sulfoxide verwenden. Als besonders bevorzugte Lösungsmittel seien jedoch Ethanol, Ethylenglykolmonomethylether, Ethylenglykolmonoethylether, Butanol oder Dimethylformamid erwähnt. Die Auswahl wird im allgemeinen dadurch festgelegt, daß das gewünschte Reaktionsprodukt der allgemeinen Formel I hinreichend wenig löslich ist und aus der Reaktionsmischung auskristallisiert.

Als basische Kondensationsmittel werden Natriumhydroxid, Kaliumhydroxid, Natriumhydrid, Natriumamid, insbesondere aber Alkalialkoholate, wie Natriummethylat, Natriumethylat oder Kalium-t-butanolat,

verwendet.

Die Umsetzung der Reaktanden erfolgt vorzugsweise in einem Alkohol in Gegenwart des entsprechenden Alkalialkoholates.

Die Stöchiometrie der Reaktanden läßt sich in weiten Grenzen variieren. Zur Herstellung von erfindungsgemäßen Isochinolinen, in denen n gleich 1 ist, wird die Aminomethylverbindung der Formel III im allgemeinen in einem 1,5- bis 5-fachen Überschuß gegenüber dem Diketon der Formel II eingesetzt, während bei der Herstellung von Isochinolinen, in denen n gleich 2 ist, das Diketon der Formel II in einem zweifachen Überschuß gegenüber der Aminomethylverbindung der Formel III eingesetzt wird. Das stöchiometrische Verhältnis zwischen Base und Aminomethylverbindung der Formel III wird durch die Stärke der eingesetzten Base beeinflußt, so daß hier ein 1,1- bis 10-facher Überschuß der Base verwendet wird.

Das Verhältnis von Lösungsmittel zur Summe der Reaktanden kann erfindungsgemäß ebenfalls in einem sehr weiten Bereich variiert werden. Im allgemeinen arbeitet man so, daß ein Gewichtsverhältnis von 5:1 bis 50:1 von Lösungsmittel zur Summe von Diketon der allgemeinen Formel II und Aminomethylverbindung der allgemeinen Formel III Verwendung findet.

Das erfindungsgemäße Verfahren kann in einem verhältnismäßig breiten Temperaturbereich von etwa 60 °C bis 200 °C durchgeführt werden, wobei der Bereich zwischen 80 und 140 °C bevorzugt wird.

Die Reaktionsdauer liegt je nach Art der Reaktanden, der eingesetzten Base und der Temperatur bei 2 bis 24 Stunden. Eine Reaktionsdauer von 4 bis 5 Stunden ist jedoch bevorzugt.

Im allgemeinen wird das erfindungsgemäße Herstellungsverfahren so geführt, daß zunächst das basische Kondensationsmittel im Lösungsmittel vorgelegt und dann das Diketon der Formel II und die Aminomethylverbindung der Formel III hinzugefügt werden. Bei der Verwendung luftempfindlicher Aminomethylverbindungen der Formel III wird unter Inertgasatmosphäre, beispielsweise Stickstoff, gearbeitet. Das Gemisch wird unter Rühren auf die gewünschte Temperatur gebracht, wobei im allgemeinen klare Lösungen entstehen, die sich mit der Zeit in ihrer Farbe vertiefen. Bisweilen beginnt die Kristallisation der erfindungsgemäßen Isochinoline bereits während der Umsetzungsperiode, bleibt die Lösung jedoch klar, so wird nach der festgelegten Zeit abgekühlt, wobei die Produkte im allgemeinen auskristallisieren. Sie werden aus der Reaktionsmischung abgetrennt, getrocknet und gegebenenfalls umkristallisiert. Für die Umkristallisation haben sich weniger polare Lösungsmittel oder Lösungsmittelgemische, wie beispielsweise Toluol oder Toluol/Hexan als günstig erwiesen.

Wenn die erfindungsgemäßen Verbindungen der allgemeinen Formel I nicht oder nur unvollständig auskristallisieren, so lassen sie sich durch Zugabe eines Nichtlösers aus der Reaktionsmischung ausfällen, durch Filtration isolieren und durch Kristallisation reinigen. Alternativ kann ihre Isolierung auch durch Extraktion erfolgen.

Die Ausbeuten an Produkt sind nach diesen Verfahren im allgemeinen sehr hoch.

Die erfindungsgemäßen neuen 1,3,4-Tri-(het-)arylisochinoline der allgemeinen Formel I sind meistens schwach bis intensiv gelb gefärbte, stabile Festkörper, die bei Bestrahlung mit UV-Licht außerordentlich stark in blauen Nuancen fluoreszieren. Darüber hinaus besitzen sie ausnahmslos gute bis hervorragende Photoleitereigenschaften. Da sie eine verhältnismäßig geringe Tendenz zeigen, in Mischungen mit polymeren Bindemitteln zu kristallisieren, lassen sie sich sehr gut als photoleitfähige Verbindungen zur Herstellung von photoleitfähigen Schichten in der Elektrophotographie und Xerographie, etwa im Bereich des Flachdrukkes und der Resisttechnik, verwenden.

Die nachstehenden Beispiele sollen die Erfindung erläutern. Der Erfindungsgedanke ist jedoch nicht auf diese Ausführungen beschränkt. Prozente sind immer Gewichtsprozente, Schmelz- und Siedepunkte sind, sofern nicht anders angegeben, unkorrigiert.

Da die aus der Literatur bekannten Synthesen für die Ausgangsprodukte nach der allgemeinen Formel II im allgemeinen sehr aufwendig und zeitraubend sind, wird im folgenden zunächst eine alternative Darstellungsmöglichkeit für zahlreiche Diketone der Formel II gegeben.

Beispiel 1

Herstellung von 1,2-Bis(4-diethylaminobenzoyl)benzol:

Zu 1000 ml trockenen Schwefelkohlenstoffs werden unter kräftigem Rühren 335 g (2,5 mol) Aluminiumchlorid gefügt und die Mischung auf ca. 10 °C gekühlt. Aus einem Tropftrichter werden innerhalb von etwa 30 Minuten 560 g (3,75 mol) Diethylanilin zugetropft, wobei die Temperatur mittels Eiskühlung unterhalb 15 °C gehalten wird. Der Tropftrichter wird mit 50 ml Schwefelkohlenstoff gespült. Die intensiv grünfarbige Mischung wird auf 0 °C gekühlt, und mittels des Tropftrichters eine Lösung von 132 g (0,65 mol) Phthalsäuredichlorid in 200 ml Schwefelkohlenstoff so zudosiert, daß die Temperatur beibehalten wird. Nach

beendeter Zugabe wird langsam auf Raumtemperatur erwärmen gelassen und dann noch 1 Stunde lang am Rückfluß erhitzt. Die Mischung läßt man abkühlen und versetzt sie vorsichtig mit ca. 300 ml Nitromethan. Schließlich wird auf 0 °C abgekühlt. Der nunmehr gelöste Keton-Aluminium-Komplex wird vorsichtig durch langsame Zugabe von 500 g Eis zersetzt. Nach vollständiger Zersetzung werden die Lösungsmittel und überschüssiges Diethylanilin durch Wasserdampfdestillation abgetrennt, wobei aus der wäßrigen Lösung das Keton auskristallisiert. Die Kristallisation wird über Nacht vervollständigt, das Produkt abgesaugt und mit wäßrigem Ethanol gewaschen. Nach vollständigem Trocknen wird das Produkt in Methylenchlorid aufgenommen und über neutralem Aluminiumoxid filtriert. Nach dem Einengen der intensiv gelben Lösung wird aus Ethanol umkristallisiert.

Ausbeute: 203 g (72 % d. Th.), schwach gelbliche Kristalle.

| Elementaranalyse : $C_{28}H_{32}N_2O_2$ | ber.: gef.: | C 78,4 %, C 78,6 %, | H 7,52 %, H 7,8 %, | N 6,54 %, N 6,6 % |
|---|---|---|---|---|
| Fp.: 172,5 bis 173,5 °C UV (MeOH): 248, 363 nm. | | | | |

Beispiel 2

Herstellung von 1,4-Bis(4-dimethylaminophenyl)-3-phenyl-isochinolin:

In 200 ml Ethylenglykolmonomethylether werden unter Rühren 3,4 g (148 mmol) Natrium aufgelöst. Zu der klaren Lösung werden nacheinander 12,5 g (33 mmol) 1,2-Bis(4-dimethylaminobenzoyl)benzol und 14,4 g (135 mmol) Benzylamin hinzugefügt und am Rückfluß erhitzt. Nach ca. 3 Stunden beginnt die Kristallisation eines gelben Pulvers. Die Mischung wird weitere zwei Stunden erhitzt und zur Vervollständigung der Kristallisation nach Abkühlen einige Zeit stehengelassen. Das Produkt wird abfiltriert, mit wäßrigem Ethanol nachgewaschen und getrocknet. Das so erhaltene Rohprodukt weist einen Schmelzintervall von 245 bis 249 °C auf. Zur Reinigung wird aus Toluol/Ethanol umkristallisiert.
Ausbeute: 12,7 g (85 % d. Th.), schwach gelbliche, intensiv blau fluoreszierende Kriställchen.

| Elementaranalyse: $C_{31}H_{29}N_3$ | ber.: gef.: | C 83,93 %, C 84,0 %, | H 6,59 %, H 6,8 %, | N 9,4 % N 9,4 % |
|---|---|---|---|---|
| Fp.: 251 bis 253 °C UV (MeOH): 281,375 nm. | | | | |

Beispiel 3

Herstellung von 1 ,4-Bis(4-diethylaminophenyl)-3-(4-aminomethylphenyl)-isochinolin:

In 60 ml Ethylenglykolmonomethylether werden 1,15 g (50 mmol) Natrium gelöst. Dazu werden 4,29 g (10 mmol) des in Beispiel 1 beschriebenen Diketons und 5,45 g (40 mmol) 4-Aminomethylbenzylamin gefügt und 5 Stunden am Rückfluß erhitzt. Nach einiger Zeit beobachtet man die Kristallisation einer geringen Menge einer gelben Verbindung. Nach dem Abkühlen erfolgt keine weitere Kristallisation und man filtriert das gelbe Pulver ab. Sein Gewicht beträgt 0,4 g, und es wird als das dimere Isochinolin (vgl. Beispiel 4) identifiziert. Das gelbe Filtrat wird mit Wasser versetzt und das ölig anfallende Produkt durch Abdekantieren der Lösung isoliert. Durch Digerieren in Ethanol wird das Produkt semikristallin. Die Rohausbeute beträgt 4,1 g (77 % d. Th.). Das Produkt wird aus Methanol umkristallisiert.

Gelbe Kristalle.

| Elementaranalyse: $C_{36}H_{40}N_4$ | ber.: gef.: | C 81,78 %, C 81,9 %, | H 7,62 %, H 7,7 %, | N 10,59 %. N 10,4 % |
|---|---|---|---|---|
| Fp.: 142 bis 143 °C UV (MeOH): 276, 375 nm. | | | | |

Beispiel 4

Herstellung von 1,4-Bis-[3-(1,1',4,4'-tetrakis-(4-diethylaminophenyl))-isochinolinyl]-benzol:

In 100 ml Ethylenglykolmonomethylether werden 2,3 g (100 mmol) Natrium gelöst. Dazu werden 7,5 g (18 mmol) des in Beispiel 1 beschriebenen Diketons und 1,2 g (9 mmol) 4-Aminomethylbenzylamin gegeben und die Mischung 16 Stunden am Rückfluß erhitzt. Nach Abkühlen wird das ausgefallene gelbe Produkt abfiltriert und aus Toluol umkristallisiert.

Ausbeute: 4,3 g (46 % d. Th.), gelbe Kristalle.

| Elementaranalyse: $C_{64}H_{68}N_6$ | ber.: gef.: | C 83,43 %, C 83,3 %, | H 7,44 %, H 7,7 %, | N 9,12 %. N 9,1 %. |
|---|---|---|---|---|
| Fp.: 266 bis 267 °C UV (MeOH): 275, 367 nm | | | | |

Beispiele 5 bis 37

Die nachstehend beschriebenen Verbindungen wurden analog der Umsetzung in Beispiel 2 hergestellt, wobei im Einzelfall lediglich geringfügige Änderungen der Reaktionsdauer, des Verhältnisses Reaktanden zu Lösungsmittel und der Aufarbeitung durchgeführt wurden. Die mit einem Stern versehenen Beispiele (*) wurden analog Beispiel 4 hergestellt. Die mit (**) versehenen Beispiele ergaben isomere Produktgemische, die nicht weiter aufgetrennt wurden.

| Beispiel Nr. | n | $R_1$ | $R_2$ | $R_3$ | Fp (°C) |
|---|---|---|---|---|---|
| 5 | 1 | 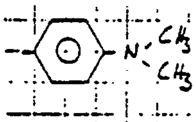 | 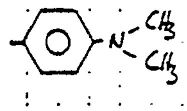 | 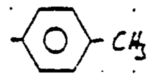 | 220-222°C Toluol/Hexan |
| 6 | 1 | 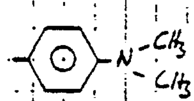 | 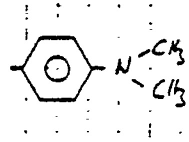 | 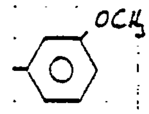 | 194-195°C Dimethylformamid (DMF)/$H_2O$ |
| 7 | 1 | 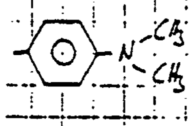 | 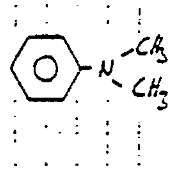 | 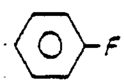 | 230-231,5°C Toluol/n-Hexan |
| 8 | 1 | 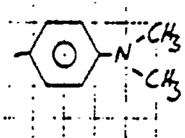 | 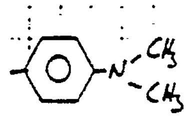 | 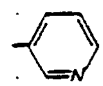 | 236-239°C Toluol/n-Hexan |
| 9 | 1 | 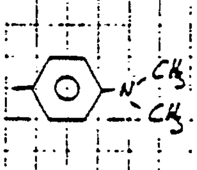 | 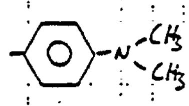 | 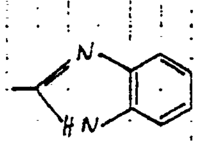 | 345-347°C DMF |
| 10 ** | 1 | 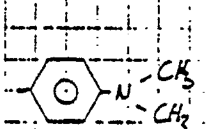 | 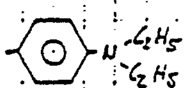 | 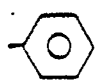 | 192-213°C Toluol/n-Hexan |
| 11 | 1 | 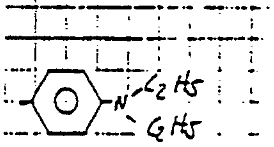 | 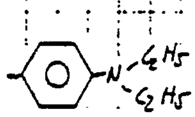 | 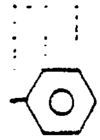 | 142-143°C Ethanol |
| 12 | 1 | 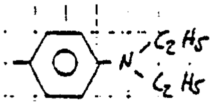 | 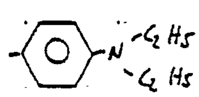 | 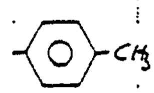 | 158°C Toluol/n-Hexan |

| Beispiel Nr. | n | $R_1$ | $R_2$ | $R_3$ | Fp (°C) |
|---|---|---|---|---|---|
| 13 | 1 | 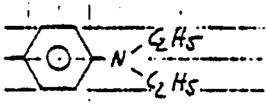 | 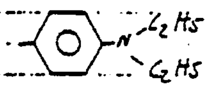 | 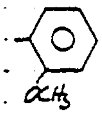 | 151,5-152,5°C Toluol/n-Hexan |
| 14 | 1 | 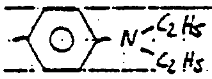 | 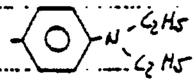 | 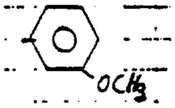 | 141,5-143°C Toluol/n-Hexan |
| 15 | 1 | 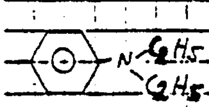 | 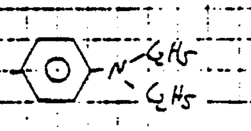 | 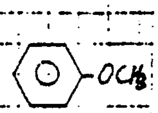 | 195-196,5°C Toluol/n-Hexan |
| 16 | 1 | 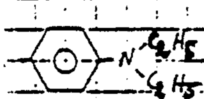 | 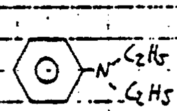 | 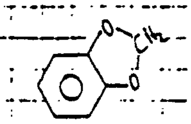 | 179-180,5°C Toluol/n-Hexan |
| 17 | 1 | 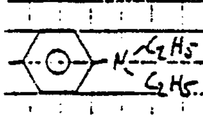 | 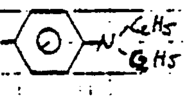 | 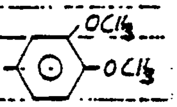 | 144-145°C Toluol/n-Hexan |
| 18 | 1 | 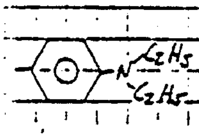 | 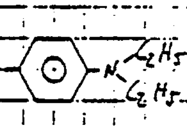 | 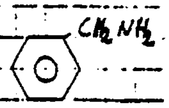 | 138-138,5°C n-Hexan |
| 19* | 2 | 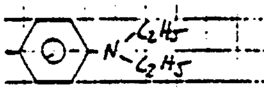 | 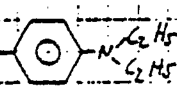 | 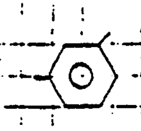 | 216-217°C Toluol/n-Hexan |
| 20 | 1 | 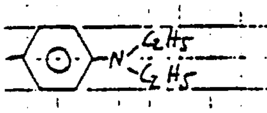 | 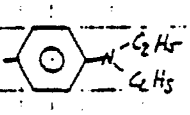 | 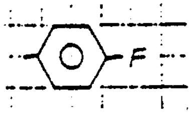 | 145-146°C Toluol/n-Hexan |

| Beispiel Nr. | n | $R_1$ | $R_2$ | $R_3$ | Fp (°C) |
|---|---|---|---|---|---|
| 21 | 1 | 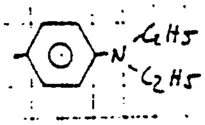 | 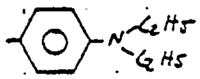 | 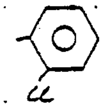 | 166-167°C Toluol/n-Hexan |
| 22 | 1 | 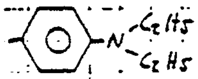 | 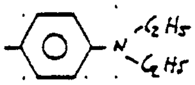 | 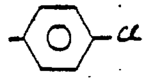 | 149-150°C Toluol/n-Hexan |
| 23 | 1 | 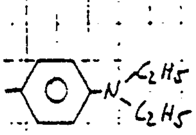 | 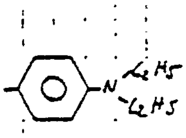 | 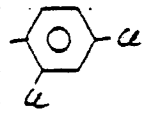 | 194-195°C Toluol/n-Hexan |
| 24 | 1 | 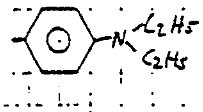 | 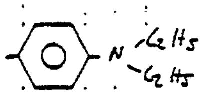 | 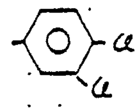 | 167-169°C Toluol/n-Hexan |
| 25 | 1 | 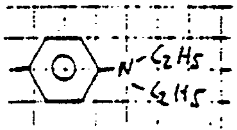 | 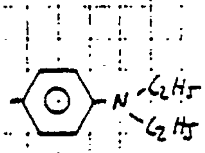 | 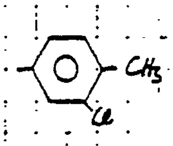 | 148-149°C Toluol/n-Hexan |
| 26 | 1 | 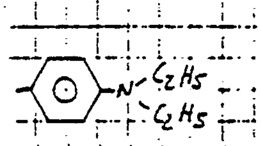 | 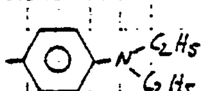 | 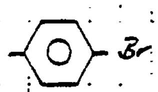 | 162-164°C Toluol/n-Hexan |
| 27 | 1 | 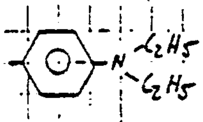 | 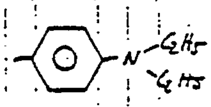 | 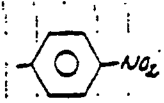 | 188,5-189°C DMF |
| 28 | 1 | 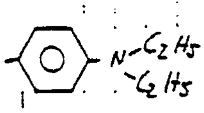 | 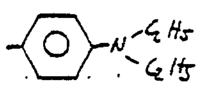 | 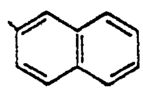 | 209-210°C Toluol |

| Beispiel Nr. | n | $R_1$ | $R_2$ | $R_3$ | Fp (°C) |
|---|---|---|---|---|---|
| 29 | 1 | 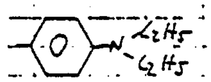 | 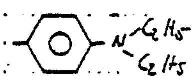 | 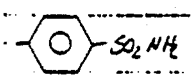 | 226-228°C Methanol/ $H_2O$ |
| 30 | 1 | 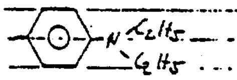 | 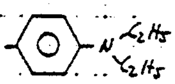 | 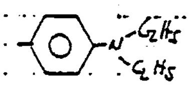 | 163-164,5°C Toluol/n-Hexan |
| 31 (Bezugsbeispiel) | 1 | 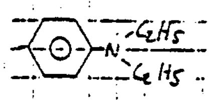 | 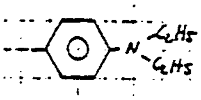 | 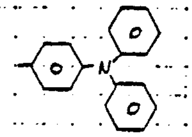 | 223,5-225°C Toluol/n-Hexan |
| 32 | 1 | 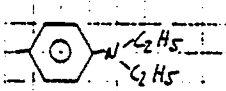 | 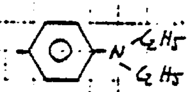 | 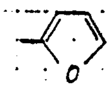 | 205-206,5°C Toluol/ n-Hexan |
| 33 | 1 | 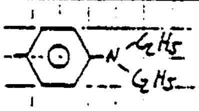 | 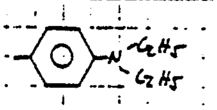 | 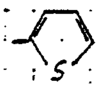 | 218-218,5°C Toluol/n-Hexan |
| 34 | 1 | 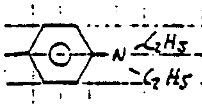 | 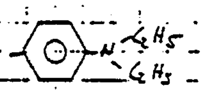 | 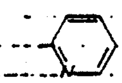 | 191-192,5°C Toluol/ n-Hexan |
| 35 | 1 | 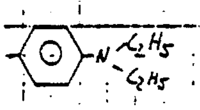 | 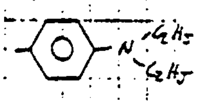 | 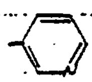 | 157-158°C Toluol/n-Hexan |
| 36 | 1 | 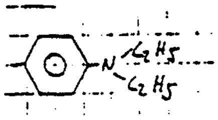 | 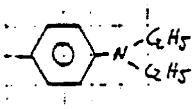 | 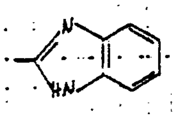 | 207°C DMF/$H_2O$ |

| Beispiel Nr. | n | $R_1$ | $R_2$ | $R_3$ | Fp (°C) |
|---|---|---|---|---|---|
| 37** (Bezugsbeispiel) | 1 | 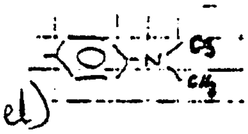 | 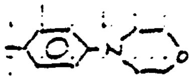 | 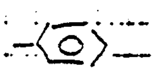 | 167-183°C Toluol/n-Hexan |

Beispiel 38

Entsprechend Beispiel 1 wird ein Diketon nachstehender Formel hergestellt, indem man Julolidin mit Phthalsäuredichlorid umsetzt. Das Produkt hat die Struktur

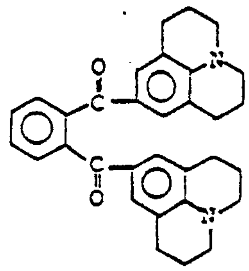

und zersetzt sich beim Erwärmen unter Dunkelfärbung ohne definierten Schmelzpunkt.

| | | | | |
|---|---|---|---|---|
| Elementaranalyse: | gef.: | C 80,4 %, | H 6,9 %, | N 5,9 %. |
| $C_{32}H_{32}N_2O_2$ | ber.: | C 80,64 %, | H 6,77 %, | N 5,88 %. |

10 mmol der vorliegenden Verbindung werden in einer Lösung aus 50 ml Ethylenglykolmonomethylether und 50 mmol Natrium unter Luft- und Feuchtigkeitsausschluß gelöst und mit 40 mmol Benzylamin versetzt und zum Rückfluß erhitzt. Nach 4 Stunden wird erkalten gelassen, das nach längerem Stehen ausgefallene Produkt abfiltriert und mit Ethanol gewaschen.

Man erhält 4,87 g, entsprechend 89 % d. Th., eines gelben Pulvers, welches sich bei längerem Stehen an der Luft rötlich verfärbt und bei der Schmelzpunktbestimmung bis 350 °C unter Dunkelfärbung und Anzeichen von Zersetzung nicht schmilzt. Das Chromatogramm (Laufmittel: Toluol/Essigsäurebutylester 3:1; Kieselgel) zeigt eine einheitliche, intensiv grün-bläulich fluoreszierende Verbindung nachstehender Formel

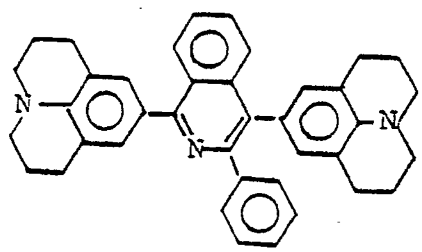

| | | | | |
|---|---|---|---|---|
| Elementaranalyse: | gef.: | C 85,7 %, | H 6,5 %, | N 7,8 %. |
| $C_{39}H_{37}N_3$ | ber.: | C 85,52 %, | H 6,81 %, | N 7,67 %. |

Obwohl zahlreiche elektrophotographische Verfahren, dafür benötigte Materialien und Varianten für den Aufbau von Aufzeichnungsmaterialien bekannt sind, besteht wegen der gestiegenen Anforderungen an die Reproduktionssysteme nach wie vor ein Bedarf an neuen, hochempfindlichen Photoleitern.

Die entsprechend den Herstellungsbeispielen 2 bis 38 synthetisierten Isochinolinderivate zeigen hervorragende Eigenschaften als photoleitfähige Verbindungen ("Ladungstransportverbindungen"), so daß sie in hochempfindlichen elektrophotographischen Aufzeichnungsmaterialien, beispielsweise gedruckten Schaltungen oder Flachdruckformen, aber auch für Materialien in cyclisch arbeitenden Verfahren, z.B. Kopiergeräten, vorteilhaft einsetzbar sind.

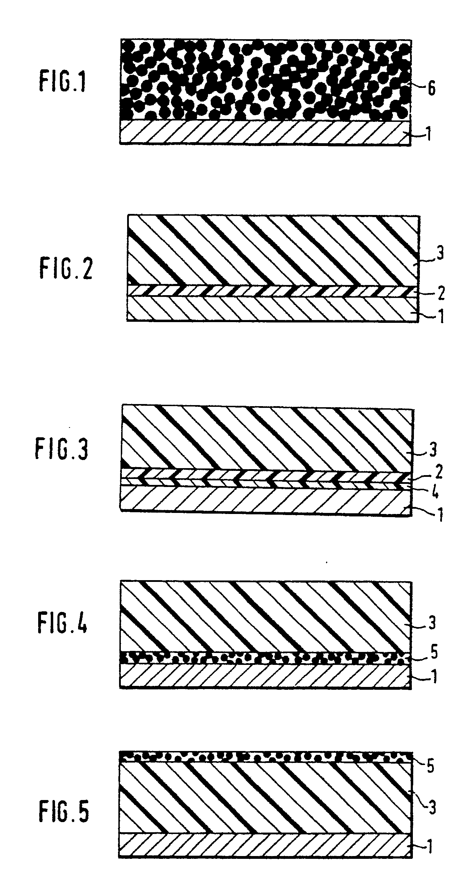

Die erfindungsgemäßen photoleitfähigen Verbindungen können mit Vorteil sowohl in einschichtig als auch mehrschichtig angeordneten Aufzeichnungsmaterialien verwendet werden, die anhand der beigefügten Figuren 1 bis 5 schematisch näher erläutert werden.

Position 1 zeigt jeweils den elektrisch leitenden Schichtträger an, Position 2 weist auf die Ladungsträger erzeugende Schicht hin, und Position 3 gibt die den Photoleiter (Ladungstransportverbindung) enthaltende Ladungstransportschicht an. Mit Position 4 wird eine isolierende Zwischenschicht bezeichnet. Position 5 gibt eine Schicht wieder, die eine Ladungsträger erzeugende Schicht in Dispersion mit einem Bindemittel darstellt. Mit Position 6 ist eine photoleitfähige Schicht aus erfindungsgemäßer, photoleitfähiger Verbindung, homogen oder dispers vorliegender Ladungen erzeugender Verbindung und Bindemittel usw. bezeichnet.

Beim Einsatz elektrophotographischer Aufzeichnungsmaterialien für Flachdruckformen werden einschichtige Systeme, in denen die Schicht aus Bindemittel, Photoleiter und Ladungsträger erzeugender Verbindung besteht, bevorzugt (Figur 1). Im allgemeinen wird dabei eine Lösung oder Dispersion der Komponenten in einem organischen Lösungsmittel auf den Schichtträger so aufgebracht, daß nach Trocknung eine Schicht mit einer Dicke von 0,5 bis 40 µm zurückbleibt. Bevorzugt sind Schichtdicken von 2 bis 15 µm.

Wird das Aufzeichnungsmaterial im Kopiersektor eingesetzt, so sind mehrschichtige Anordnungen günstig (Figuren 2 bis 5), wobei die Ladungsträger erzeugende Schicht 2 bzw. 5 bevorzugt weniger als 0,8 µm, die den Photoleiter enthaltende Schicht 3 im allgemeinen zwischen 2 und 30 µm beträgt.

Eine zusätzliche Verbesserung der elektrophotographischen Eigenschaften kann durch Einführung einer isolierenden Zwischenschicht 4, welche als Sperrschicht oder haftvermittelnd wirkt, erreicht werden (Figur 3). Je nach Einsatz kann ihre Dicke zwischen 10 nm bis 2 µm betragen.

Als leitende Schichtträger lassen sich prinzipiell alle elektrisch leitenden Materialien verwenden, die flächig beschichtbar sind.

Bei Verwendung als Flachdruckform sind beispielsweise Schichtträger in 80 bis 500 µm Stärke aus Aluminium, Zink, Magnesium oder Kupfer oder sogenannte Mehrmetallplatten geeignet, die je nach Verwendung durch geeignete Maßnahmen vorbehandelt, aufgerauht und anodisiert sein können. Neben Aluminiumblechen und -folien sind auch Kunststoffolien mit metallisierter oder metallbedampfter Oberfläche, z.B. aluminiumbedampftes Polyethylen, aluminiumbedampfter Polyester oder elektrisch leitendes Spezialpapier bevorzugt.

In der Xerographie finden derartige Schichtträger auch in Form von Trommeln oder flexiblen Endlosbändern Verwendung.

Obwohl einige der erfindungsgemäßen Photoleiter selbst filmbildend sind, ist es häufig günstig, ein polymeres Bindemittel zur Verbesserung der Schichteigenschaften zuzufügen. Die Wahl des geeigneten Bindemittels ist abhängig vom Verwendungszweck des Aufzeichnungsmaterials. Im Kopiersektor sind dies sowohl für die Ladungstransport- als auch Ladungserzeugungsschicht im besonderen Celluloseester, Polyester, Polyvinylchloride, Polyvinylacetate, Polycarbonate, Acryl- und Methacrylharze und Copolymere, wie z.B. Styrol-Maleinsäureanhydrid-, Vinylchlorid-Maleinsäureanhydrid- oder Styrol-Buadien-Copolymere, wobei insbesondere ihre Filmbildungs- und Haftungseigenschaften sowie die elektrischen Eigenschaften eine entscheidene Rolle spielen.

Für Flachdruckformen und gedruckte Schaltungen sind besonders solche Bindemittel geeignet, die in sauren oder bevorzugt alkalischen, wäßrig-alkoholischen oder wäßrigen Lösungsmittelgemischen löslich sind. Neben guter Löslichkeit sind hier Filmbildungs- und Haftungseigenschaften, Druckdauerhaftigkeit und physiologische Unbedenklichkeit entscheidend.

Vorteilhafterweise werden hochmolekulare Bindemittel mit ausreichender Alkalilöslichkeit verwendet. Die Alkalilöslichkeit läßt sich durch Einbau gewisser Gruppen, wie beispielsweise Säureanhydridgruppen, Carboxylgruppen, phenolische und aliphatische Hydroxygruppen, Sulfonsäure-, Sulfonamid- oder Sulfonimidgruppen oder durch elektronenziehende Gruppen aktivierte Urethangruppen erreichen.

Für die Herstellung von lichtempfindlichen Schichten für den Drucksektor sind Copolymerisate mit Säureanhydridgruppen, teilveresteren Säureanhydridgruppen, Carboxylgruppen und Phenolharzen besonders geeignet, da derartige Mischungen hervorragende elektrophotographische Eigenschaften und sehr gute drucktechnische Eigenschaften miteinander verbinden.

Insbesondere seien folgende Polymerisate erwähnt: Copolymerisate aus Styrol oder substituierten Styrolen mit Maleinsäureanhydrid, Copolymerisate aus Styrol oder substituiertem Styrol mit teilverestertem Maleinsäureanhydrid, Copolymerisate aus Acrylsäure, Methacrylsäure und Acylsäureestern sowie Umsetzungsprodukte aus freie Hydroxygruppen enthaltenden Polyvinylacetalen und Sulfonylisocyanaten.

Werden Phenolharze als Bindemittel eingesetzt, so werden Homo- oder Copolymerisate des Hydroxystyrols oder Novolakharze bevorzugt, wobei letztere beispielsweise durch Kondenstion von Phenol oder Kresol mit Formaldehyd erhältlich sind.

In diesen Fällen erhält man Schichten, die in Entwicklerlösungen, bestehend z.B. aus 70 % Wasser, 25 % Propanol und 5 % Natriummetasilikat, hinreichend löslich sind und gleichzeitig gute elektrophotographische Eigenschaften aufweisen.

Die genannten Harze lassen sich allein oder als Gemisch mit anderen Harzen verwenden. Insgesamt sind die erfindungsgemäß verwendbaren Bindemittel nicht auf die hier beschriebenen Harze beschränkt.

Wenngleich die erfindungsgemäßen Verbindungen von sich aus photoleitfähig, d.h. ladungserzeugend und ladungstransportierend wirken, so läßt sich die Lichtempfindlichkeit eines sie enthaltenden elektrophotographischen Aufzeichnungsmaterials gegenüber sichbarem Licht, insbesondere in speziell interessanten spektralen Bereichen, durch den Zusatz von sensibilisierenden Farbstoffen oder Pigmenten erheblich erhöhen.

Bei einschichtigen Systemen können Farbstoffe aus der Triarylmethanreihe und/oder Xanthen-, Oxazin-, Thiazin-, Cyanin-, Pyrylium-, Chinacridonfarbstoffe oder indigoide Farbstoffe, wie z.B. Rhodamin FB (C.I. 45160), Malachitgrün (C.I. 42000), Methylviolett (C.I. 42535), Kristallviolett (C.I. 42555), Brilliantgrün (C.I. 42040), Methylenblau (C.I. 52015), Methylengrün (C.I. 52020), Caprylblau (C.I. 48035), Astrazongelb (C.I. 48035), Astrazonorange (C.I. 48040), Astrazonrot (C.I. 48020) Aizen Astra Phloxin (C.I. 48070) oder deren Mischungen, oder anorganische oder organische Pigmente, wie Zinkoxid, Zinksulfid oder Phthalocyaninpigmente, Azopigmente, Perylenpigmente, Benzimidazol- und Indanthronpigmente, z.B. Kupferphthalocyanin in seinen verschiedenen Kristallformen, N,N-Dialkylperylentetra-carbonsäurediimide oder Chlordiane Blau Verwendung finden.

Die Ladungsträger erzeugenden Substanzen werden zusammen mit der photoleitfähigen Verbindung, Bindemittel und gegebenenfalls weiteren Zusätzen in einem organischen Lösungsmittel gelöst oder dispergiert und dann nach bekannten Verfahren, z.B. Rakeln, auf den Schichtträger aufgebracht und getrocknet.

Bei mehrschichtigen Systemen können neben den oben bezeichneten Farbstoffen und Pigmenten beispielsweise Selen und dessen Legierungen sowie Ladungsübertragungs-und eutektische Komplexe Einsatz finden. Auch in diesen Fällen wird die Ladungsträger erzeugende Substanz häufig zusammen mit einem Bindemittel und sonstigen Zusätzen in einem organischen Lösungsmittel gelöst oder dispergiert und durch Rakeln aufgetragen; sie kann aber auch durch Sublimation oder verwandte Aufdampftechniken auf den Schichtträger aufgebracht werden.

Des weiteren können als Zusätze den einzelenen Schichten Verlaufmittel, Weichmacher oder Haftvermittler zugesetzt werden.

Die erfindungsgemäßen photoleitfähigen Verbindungen können auch in Kombination mit anderen, bekannten Photoleitern verwendet werden. Das Mischungsverhältnis mit dem Bindemittel kann in weiten Grenzen den Erfordernissen entsprechend variiert werden, jedoch sind durch die Forderung nach maximaler Lichtempfindlichkeit bestimmte Grenzen gesetzt. Es hat sich ein Mischungsverhältnis von etwa 1:1 Gewichtsteilen als günstig erwiesen, vorteilhafterweise lassen sich aber auch Verhältnisse zwischen 4:1 und 1:2 verwenden.

Die Ladungsträger erzeugende Substanz wird erfindungsgemäß bevorzugt zwischen 0,01 und 30 %, bezogen auf das Gewicht der photoleitfähigen Verbindung, eingesetzt. Als besonders günstig haben sich Mengen zwischen 0,5 und 15 % erwiesen. Beim Einsatz von Pigmenten können höhere Konzentrationen angewendet werden, ohne daß dadurch die elektrophotographischen Eigenschaften des Aufzeichnungsmaterials negativ beeinflußt werden.

Wenngleich homogene Schichten oder Monodispersionsschichten gemäß Figur 1 generell die geringste Lichtempfindlichkeit aufweisen, so haben sie doch gegenüber den Mehrfachschichten den Vorteil, daß sie sowohl positiv als auch negativ aufladbar sind, während letztere in den Anordnungen nach den Figuren 2, 3, 4 negativ, in inverser Anordnung nach Figur 5 positiv aufladbar sind.

Für eine Beurteilung der Photoleitfähigkeit der erfindungsgemäßen Isochinoline ist es unerheblich, welche Schichtanordnung vorliegt; grundsätzlich kann ihre Verwendung in allen Anordnungen gemäß Figuren 1 bis 5 erfolgen.

Beispiel 39

Eine Beschichtungslösung, bestehend aus

| | |
|---|---|
| 30,0 g | eines Copolymerisates von Styrol und Maleinsäureanhydrid mit einem mittleren Molekulargewicht von etwa 80.000, |
| 20,0 g | der Verbindung Nr. 11, |
| 0,1 g | Rhodamin FB (C.I. 45170) |
| 0,6 g | Astrazonorange R (C.I. 48040) in |
| 220,0 g | Tetrahydrofuran, |
| 140,0 g | Ethylenglykolmonomethylether und |
| 44,0 g | Butylacetat, |

wird auf eine 0,3 mm starke, elektrochemisch aufgerauhte und mit Polyvinylphosphonsäure nachbehandelte Aluminiumfolie mittels einer Rakel derart aufgetragen, daß nach dem Verdunsten des Lösungsmittelgemisches eine photoleitfähige Schicht mit einem Gewicht von 5,8 g/m² zurückbleibt.

Die Schicht wird mit einer Corona auf -450 V aufgeladen und in einer Reprokamera mit 10 Halogenstrahlern zu je 600 W 15 sec lang belichtet. Als Vorlage dient eine Klebemontage, die die üblichen Prüfelemente enthält.

Nach der Entwicklung des durch Belichtung entstandenen latenten Ladungsbildes mit einem handelsüblichen Trockenentwickler und dessen thermischer Fixierung erhält man ein sauberes, grundfreies und randscharfes Abbild der Vorlage.

Zur Umwandlung in eine Druckform bringt man die Aluminiumfolie mit der das fixierte Tonerbild enthaltenden Photoleiterschicht in eine Küvette, die eine Entschichterlösung enthält, welche aus 50 g Natriumsilikat, 250 g Glycerin (80 %ig), 390 g Ethylenglykol und 310 g Methanol hergestellt wurde.

Die entschichtete Druckplatte wird mit Wasser gespült und abgerakelt. An den entschichteten Stellen erscheint das Schichtträgermaterial, ohne Reste eines Farbschleiers zu zeigen.

Nach Einspannen der Druckform in eine Offsetdruckmaschine erhält man mehrere zehntausend qualitativ hochwertige Drucke.

Beispiel 40

Ein wie in Beispiel 39 hergestelltes Aufzeichnungsmaterial wird in einem Dyntest-Gerät vermessen. Die in diesem Gerät verwendete Glühfadenlampe hat eine Temperatur von 2800 °K. Die Schicht wird auf -500 V aufgeladen und die Dunkelentladung aufgezeichnet. Nach 1 min beträgt das Restpotential $U_D$ -428 V, entsprechend 85,5 % der Anfangsaufladung.

Wird die Schicht auf -500 V aufgeladen und anschließend belichtet, so wird nach 1 min ein Restpotential $U_H$ von -9 V, entsprechend 1,8 % der Anfangsaufladung, erhalten. Die Schicht ist nach 12,8 sec auf $E_{1/10}$ (-50 V) entladen, die Halbwertsempfindlichkeit $E_{1/2}$ beträgt 12,2 µJ/cm².

Beispiele 41 bis 44

Es werden Beschichtungslösungen entsprechend Beispiel 39 hergestelllt, wobei anstelle der Verbindung Nr. 11 die Verbindungen Nr. 12, 16, 20 und 30 verwendet werden. Entsprechend Beispiel 40 kommt man zu folgenden Ergebnissen:

| Verbindung Nr. | $U_D$ (V) | $U_H$ (V) | $E_{1/2}$ (µJ/cm²) |
|---|---|---|---|
| 12 | -427 (85,4%) | -8 (1,6%) | 11,0 |
| 16 | -385 (77,0%) | -10 (2,0%) | 11,3 |
| 20 | -437 (87,4%) | -16 (3,2%) | 12,5 |
| 30 | -414 (82,8%) | -6 (1,2%) | 10,3 |

Bei der Bebilderung, Betonerung und Entschichtung in einem handelsüblichen Gerät ergeben alle in den Beispielen 41 bis 44 verwendeten Aufzeichnungsmaterialien Druckformen hoher Qualität, die sich durch rasche Verarbeitbarkeit, hohe Volltondeckuung, gute Randschärfe und Grundfreiheit auszeichnen.

Beispiel 45

In der in Beispiel 39 beschriebenen Beschichtungslösung wird die Verbindung Nr. 11 durch die Verbindung 2,5-Bis-(4-diethylaminophenyl)-oxdiazol-1,3,4 (DE-PS 10 58 875, entsprechend US-PS 3,189,447) ersetzt. Man findet folgende Ergebnisse:
$U_D$ (V) = -382 V (76,4 %) $U_H$ (V) = -11 V (2,2 %)
$E_{1/2}$ = 13,6 µJ/cm².

Wie ersichtlich, zeigen die erfindungsgemäßen Photoleiter Empfindlichkeiten, die im Bereich des häufig kommerziell verwendeten Oxdiazolderivats liegen. Insgesamt weisen sie allerdings eine deutlich geringere Dunkelentladung auf als das Oxdiazolderivat.

Beispiel 46

Auf eine aluminiumbedampfte Polyesterfolie wird N,N'-Dimethylperylen-3,4,9,10-tetracarbonsäurediimid,

dessen spektrale Lichtabsorption von 430 bis 600 nm reicht, in einer Vakuum-Bedampfungsanlage bei ca. $10^{-8}$ bar und ca. 280 °C aufgedampft. Die homogen aufgedampfte Schicht besitzt ein Schichtgewicht von etwa 150 mg/m² und deckt den Polyesterträger vollständig ab.

Auf die Aufdampfschicht wird eine Lösung aus gleichen Gewichtsteilen der Verbindung Nr. 11 und eines Polycarbonatharzes (Makrolon® 2045, Bayer) in Tetrahydrofuran geschleudert. Die Schicht wird getrocknet und ihre Lichtempfindlichkeit wird gemäß der oben beschriebenen Charakterisierungsmethode bestimmt zu

$U_D$ (V) = -443 V (88,5 %) $U_H$ (V) = praktisch Null

$E_{1/2}$ (µJ/cm²) = 1,65.

Beispiele 47 und 48

Die Versuchsanordnungen des Beispiels 46 werden derart wiederholt, daß die dort verwendete erfindungsgemäße Verbindung Nr. 11 durch die Vergleichsverbindungen 2-Phenyl-4(2'-chlorphenyl)-5-(4'-diethylaminophenyl)-oxazol-1,3 (DE-PS 11 20 875, entsprechend US-PS 3,257,203) und 2,5-Bis-(4-diethylaminophenyl)-oxadiazol-1,3,4 ersetzt wird. Die $E_{1/2}$-Werte von Schichten dieser Verbindungen betragen 2,56 und 1,76 µJ/cm² bei gleichen Schichtdicken.

**Patentansprüche**

1. 1,3,4-Tri-(het-)arylisochinoline der allgemeinen Formel I

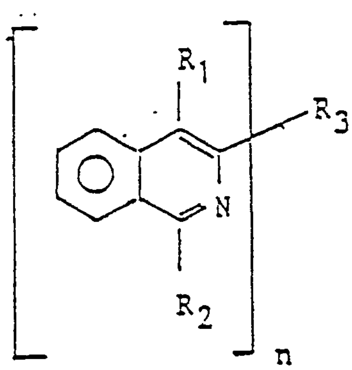

in der

$R_1$ und $R_2$ gleich oder verschieden sind und Di-($C_1$ bis $C_4$)alkylaminophenyl, Julolidyl oder Aminophenyl,

$R_3$ Phenyl, Naphthyl oder Phenylen, die gegebenfalls durch mindestens ein Halogen, eine Nitro-, Mono- oder Di($C_1$ bis $C_4$)alkylamino-, ($C_1$ bis $C_4$)Alkyl- oder -Alkoxy-, Methylendioxy-, Amino($C_1$ bis $C_4$)alkyl-oder Sulfonamido-Gruppe substituiert sind, oder Furanyl, Thienyl, Pyridyl, Julolidyl oder Benzimidazolyl

bedeuten, und

n gleich 1 oder 2 ist.

2. Verbindungen der allgmeinen Formel I nach Anspruch 1,

in der

$R_1$ und $R_2$ gleich oder verschieden sind und 4-Di($C_1$ bis $C_4$)alkylaminophenyl,

$R_3$ Phenyl oder Naphthyl, die gegebenenfalls durch mindestens ein Halogen, eine Nitro-, Mono- oder Di($C_1$ bis $C_4$)alkylamino-, ($C_1$ bis $C_4$)Alkyl-oder -Alkoxy-, Methylendioxy-, Amino($C_1$ bis $C_4$)alkyl- oder Sulfonamido-Gruppe substituiert sind, oder Furanyl, Thienyl, Pyridyl, Julolidyl oder Benzimidazolyl

bedeuten und

n gleich 1 ist.

3. Verbindungen der allgmeinen Formel I nach Ansprüchen 1 oder 2, dadurch gekennzeichnet, daß
   $R_1$ und $R_2$ 4-Dimethylaminophenyl oder 4-Diehylaminophenyl,
   $R_3$ Phenyl oder Naphthyl, die gegebenenfalls durch mindestens ein Halogen, eine ($C_1$ bis $C_4$)Alkyl-, -Alkoxy-, Methylendioxy- oder Amino($C_1$ bis $C_4$)alkyl-Gruppe substituiert sind,

   bedeuten und
   n gleich 1 ist.

4. Verfahren zur Herstellung der Verbindungen der allgemeinen Formel I gemäß Ansprüchen 1 bis 4, dadurch gekennzeichnet, daß man ein Diketon der allgemeinen Formel II

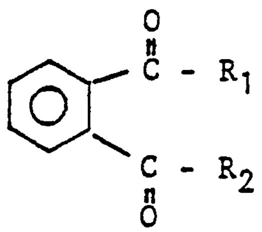

II

mit einer aromatischen oder heteroaromatischen Aminomethylverbindung der allgmeienen Formel III

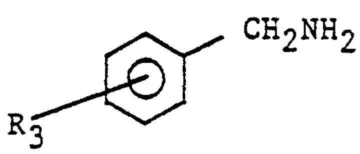

III

in denen $R_1$, $R_2$ und $R_3$ die angegebenen Bedeutungen besitzen, in einem Lösungsmittel oder Lösungsmittelgemisch unter Zusatz eines basischen Kondensationsmittels im Temperaturbereich zwischen 60 und 200 °C umsetzt, das Reaktionsprodukt abtrennt, gegebenenfalls reinigt und trocknet.

5. Verfahren nach Anspruch 4, dadurch gekennzeichnet, daß man als Lösungsmittel Ethanol, Ethylenglykolmonomethylether, Ethylenglykolmonoethylether, Butanol oder Dimethylformamid verwendet.

6. Verfahren nach Ansprüchen 4 oder 5, dadurch gekennzeichnet, daß man die Umsetzung in Gegenwart eines Alkalialkoholats durchführt.

7. Verwendung der 1,3,4-Tri-(het-)arylisochinoline nach Anspruch 1 als photoleitfähige Verbindungen.

**Claims**

1. 1,3,4-Tri-(het)aryl-isoquinolines of the general formula I

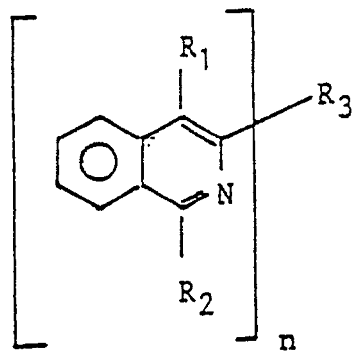

I

in which

$R_1$ and $R_2$ are identical or different and denote di($C_1$ to $c_4$)alkylaminophenyl, julolidyl or aminophenyl,

$R_3$ denotes phenyl, naphthyl or phenylene, which is optionally substituted by at least one halogen, by a nitro group, mono- or di($C_1$ to $C_4$)alkylamino group, ($C_1$ to $C_4$)alkyl group or ($C_1$ to $C_4$)alkoxy group, methylene-dioxy group, amino($C_1$ to $C_4$)alkyl group or sulfonamido group, or denotes furanyl, thienyl, pyridyl, julolidyl or benzimidazolyl, and

n denotes 1 or 2.

**2.** Compounds of the general formula I, as claimed in Claim 1, in which

$R_1$ and $R_2$ are identical or different and denote 4-di($C_1$ to $C_4$) alkylaminophenyl,

$R_3$ denotes phenyl or naphthyl which is optionally substituted by at least one halogen, a nitro group, mono- or di($C_1$ to $C_4$)-alkylamino group, ($C_1$ to $C_4$)alkyl group or ($C_1$ to $C_4$)alkoxy group, methylenedioxy group, amino($C_1$ to $C_4$)alkyl group or sulfonamido group, or denotes furanyl, thienyl, pyridyl, julolidyl or benzimidazolyl, and

n denotes 1.

**3.** Compounds of the general formula I, as claimed in any of Claims 1 and 2, in which

$R_1$ and $R_2$ denote 4-dimethyl-aminophenyl or 4-diethylaminophenyl,

$R_3$ denotes phenyl or naphthyl which are optionally substituted by at least one halogen, ($C_1$ to $C_4$)alkyl group, ($C_1$ to $C_4$)alkoxy group, methylene-dioxy group or amino($C_1$ to $C_4$)alkyl group, and

n denotes 1.

**4.** Process for the preparation of the compounds of the general formula I, as claimed in Claims 1 to 3, wherein a diketone of the general formula II

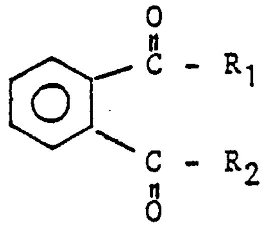

II

is reacted, at a temperature between 60 °C and 200 °C, in a solvent or solvent mixture containing a basic condensation agent, with an aromatic or heteroaromatic aminomethyl compound of the general formula III

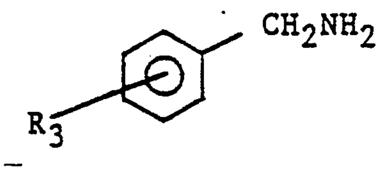

III

in which $R_1$, $R_2$ and $R_3$ have the meanings indicated above, the reaction product is separated, optionally purified, and dried.

5. The process as claimed in Claim 4, wherein ethanol, ethylene glycol monomethyl ether, ethylene glycol monoethyl ether, butanol or dimethyl formamide is used as the solvent.

6. The process as claimed in Claim 4 or 5, wherein the reaction is performed in the presence of an alkali metal alcoholate.

7. Use of the 1,3,4-tri-(het)-aryl-isoquinolines as claimed in Claim 1 as photoconductive compounds.

## Revendications

1. 1,3,4-tri-(het-)arylisoquinoléines de formule générale I

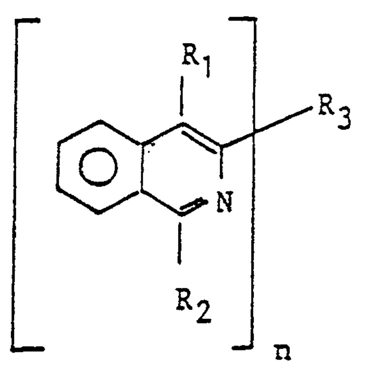

I

dans laquelle

| | |
|---|---|
| $R_1$ et $R_2$ | sont identiques ou différents et représentent un radical dialkyl($C_1$-$C_4$)-aminophényle, julolidyle ou aminophényle, |
| $R_3$ | représente un radical phényle, naphtyle ou phénylène, qui sont éventuellement substitués par au moins un substituant halogène, nitro, mono- ou dialkyl($C_1$-$C_4$)-amino, alkyle ou alcoxy en $C_1$-$C_4$, méthylènedioxy, aminoalkyle en $C_1$-$C_4$ ou sulfonamido, ou le radical furannyle, thiényle, pyridyle, julolidyle ou benzimidazolyle, et |
| n | est 1 ou 2. |

2. Composés de formule générale I selon la revendication 1, dans laquelle

| | |
|---|---|
| $R_1$ et $R_2$ | sont identiques ou différents et représentent un radical 4-dialkyl($C_1$-$C_4$)-aminophényle, |
| $R_3$ | représente un radical phényle ou naphtyle, qui sont éventuellement substitués par au moins un substituant halogène, nitro, mono- ou dialkyl($C_1$-$C_4$)-amino, alkyle ou alcoxy en $C_1$-$C_4$, méthylènedioxy, aminoalkyle en $C_1$-$C_4$ ou sulfonamido, ou le radical furannyle, thiényle, pyridyle, julolidyle ou benzimidazoyle, et |
| n | est égal à 1. |

3. Composés de formule générale I selon la revendication 1 ou 2, caractérisé en ce que

| | |
|---|---|
| $R_1$ et $R_2$ | représentent le groupe 4-diméthylaminophényle ou 4-diéthylaminophényle, |

R3 représente un radical phényle ou naphtyle, qui sont éventuellement substitués par au moins un substituant halogène, alkyle en $C_1$-$C_4$, alcoxy en $C_1$-$C_4$, méthylènedioxy ou aminoalkyle en $C_1$-$C_4$, et

n est égal à 1.

**4.** Procédé pour la préparation des composés de formule générale I selon les revendications 1 à 4, caractérisé en ce que l'on fait réagir, dans la plage de températures comprise entre 60 et 200 ° C, une dicétone de formule générale II

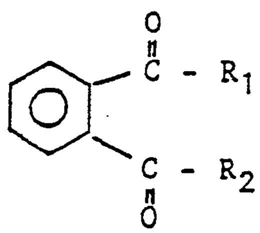

II

avec un composé aminométhyle aromatique ou hétéroaromatique de formule générale III

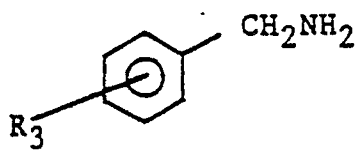

III

dans lesquelles $R_1$, $R_2$ et $R_3$ ont les significations données, dans un solvant ou mélange de solvants, avec addition d'un agent de condensation basique, on sépare le produit de réaction, éventuellement on le purifie, puis on le sèche.

**5.** Procédé selon la revendication 4, caractérisé en ce que l'on utilise en tant que solvant l'éthanol, l'éther monométhylique d'éthylèneglycol, l'éther monoéthylique d'éthylèneglycol, le butanol ou le diméthylformamide.

**6.** Procédé selon la revendication 4 ou 5, caractérisé en ce que l'on effectue la réaction en présence d'un alcoolate de métal alcalin.

**7.** Utilisation des 1,3,4-tri-(het-)arylisoquinoléines selon la revendication 1, en tant que composés photoconducteurs.

FIG.1

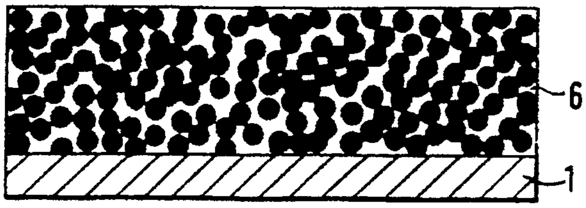


FIG.2

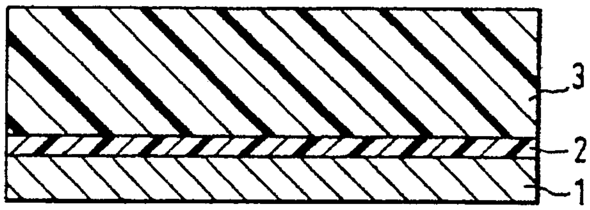


FIG.3

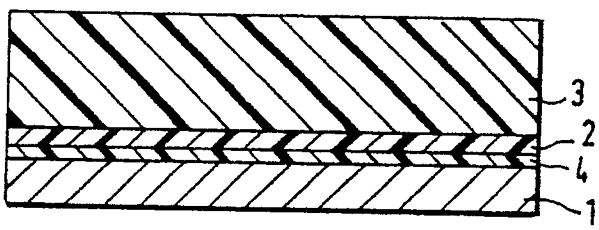


FIG.4

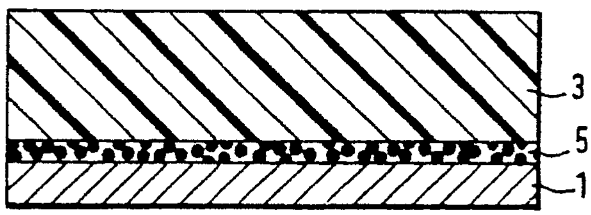


FIG.5